# EUROPÄISCHE PATENTSCHRIFT

(11) **EP 3 333 253 B1**
(45) Veröffentlichungstag und Bekanntmachung des Hinweises auf die Patenterteilung: **26.05.2021**
(21) Anmeldenummer: 17205918.0
(22) Anmeldetag: 07.12.2017
(51) Int. Cl.: C12M 1/42, C12N 5/00, C12N 1/00, C12N 13/00

(54) **AKTORSUBSTRAT, VERWENDUNG ZUR STIMULATION VON BIOLOGISCHEN ZELLEN UND VERFAHREN ZU DESSEN HERSTELLUNG**
ACTUATOR SUBSTRATE, USE FOR STIMULATING BIOLOGICAL CELLS AND METHODS OF MAKING THE SAME
SUBSTRAT D'ACTIONNEUR, UTILISATION PERMETTANT LA STIMULATION DES CELLULES BIOLOGIQUES ET SON PROCÉDÉ DE FABRICATION

(30) Priorität: 12.12.2016 DE 102016224712
(43) Veröffentlichungstag der Anmeldung: 13.06.2018
(73) Patentinhaber: Fraunhofer-Gesellschaft zur Förderung der angewandten Forschung e.V., 80686 München (DE)
(72) Erfinder: GRUNEMANN, Timo, 97070 Würzburg (DE); HEINRICH, Doris, 63875 Mespelbrunn (DE); DOMANN, Gerhard, 97204 Höchberg (DE)
(74) Vertreter: Pfenning, Meinig & Partner mbB

(56) Entgegenhaltungen:
- DE-A1-102011 010 757
- DE-A1-102011 075 127
- DE-A1-102011 107 046
- US-A1- 2012 200 200
- US-B2- 8 465 971
- C. FRIAS ET AL: "Polymeric piezoelectric actuator substrate for osteoblast mechanical stimulation", JOURNAL OF BIOMECHANICS, Bd. 43, Nr. 6, 1. April 2010 (2010-04-01), Seiten 1061-1066, XP055459971, US ISSN: 0021-9290, DOI: 10.1016/j.jbiomech.2009.12.010
- ALEXANDRE POULIN ET AL: "Dielectric elastomer actuator for mechanical loading of 2D cell cultures", LAB ON A CHIP, Bd. 16, Nr. 19, 1. Januar 2016 (2016-01-01), Seiten 3788-3794, XP055460159, ISSN: 1473-0197, DOI: 10.1039/C6LC00903D
- KARL SVENNERSTEN ET AL: "Mechanical stimulation of epithelial cells using polypyrrole microactuators", LAB ON A CHIP, Bd. 11, Nr. 19, 1. Januar 2011 (2011-01-01), Seite 3287, XP055460179, ISSN: 1473-0197, DOI: 10.1039/c1lc20436j
- SIEGFRIED BAUER ET AL: "Piezoelectric and Electrostrictive Polymers as EAPs: Devices and Applications", 1. Januar 2016 (2016-01-01), ELECTROMECHANICALLY ACTIVE POLYMERS. POLYMERS AND POLYMERIC COMPOSITES: A REFERENCE SERIES. SPRINGER, CHAM, SPRINGER INTERNATIONAL PUBLISHING, DE, PAGE(S) 533 - 547, XP009504187, ISBN: 978-3-319-31528-7 [gefunden am 2016-08-28] * Seite 535, Absatz 2 *

## Beschreibung

Die vorliegende Erfindung betrifft ein Aktorsubstrat mit einer oberen Elektrode und einer unteren Elektrode sowie einem zwischen den beiden Elektroden angeordneten und unmittelbar an die obere und untere Elektrode angrenzenden Aktorelement. Außerdem betrifft die Erfindung die Verwendung eines solchen Aktorsubstrates zur Züchtung biologischer Zellen sowie ein Verfahren zu dessen Herstellung.

Es ist mittlerweile allgemein anerkannt, dass mechanische Reize einen Einfluss auf die Funktionen und das Verhalten biologischer Zellen haben. Durch mechanische Stimulation können beispielsweise Wachstum und Differenzierung von Zellen gefördert werden. Auch Genexpressionsmuster können sich aufgrund von mechanischen Reizen ändern.

Dieser Zusammenhang wird unter anderem zur Züchtung von Zellgewebe im

Gebiet des Tissue Engineerings genutzt. Zellen und Zellgewebe können auf Trägerstrukturen angesiedelt werden, die stimuli-responsive Materialien enthalten. Durch die stimuli-responsiven Materialien können die Trägerstrukturen zu wiederholten Deformationen oder kontinuierlichen Vibrationen veranlasst werden. Solche Trägerstrukturen sind auch als Aktorsubstrate bekannt.

Im Stand der Technik sind bereits einige Aktorsubstrate auf Basis verschiedener stimuli-responsiver Materialien beschrieben, jedoch lassen sich diese Aktorsubstrate nicht mit Hilfe einfacher und industriell anwendbarer Techniken in hohen Durchsätzen produzieren.

Die DE 10 2011 010757 A1 offenbart beispielsweise Aktorsubstrate, die weiche, magnetoaktive oder elektroaktive Kompositmaterialien aus magnetisch oder elektrisch polarisierbaren Partikeln in mindestens einem Polymer als Matrix enthalten. Durch Anlegen eines äußeren Magnetfeldes oder eines elektrischen Feldes können die elastomechanischen Eigenschaften der Kompositmaterialien gesteuert werden. Es können auch hier Verformungen oder wellenartige Bewegungen an der Oberfläche des Aktorsubstrates erzeugt werden, die einen Einfluss auf angelagerte biologische Zellen ausüben. Das Aktorsubstrat und das entsprechende System zur Erzeugung des äußeren Feldes weisen aber einen so komplexen Aufbau auf, dass sie nicht schnell und mit Hilfe industrieller Methoden gefertigt werden können. Darüber hinaus kann zwar ein äußeres Feld erzeugt werden, das sich zeitlich und räumlich verändert, jedoch ist es nicht möglich ein äußeres Feld zu erzeugen, das die Oberfläche des Kompositmaterials zu einer Vibration in Form einer stehenden Welle mit mehr als zwei Knotenpunkten anregt. Außerdem zeigen die eingesetzten Kompositmaterialien keine Transparenz für sichtbares Licht, so dass das auf dem Aktorsubstrat wachsende Zellgewebe nicht mit Hilfe klassischer Lichtmikroskopiemethoden untersucht werden kann.

Auch aus der US 8465971 A1 ist eine Vorrichtung zur mechanischen Stimulation des Zellwachstums bekannt. Sie basiert auf piezoelektrischen Elementen, die in einer luftdichten Kammer platziert sind. Auf den Piezoelementen liegt ein festes Substrat auf, welches die Schwingungen der Piezoelemente an das Zellgewebe weitergibt. Nachteilig ist hier ebenfalls, dass die Vorrichtung nicht in hohen Stückzahlen in einem automatisierten Prozess hergestellt werden kann.

C. Frias et al: "Polymeric piezoelectric actuator substrate for osteoblast mechanical stimulation", JOURNAL OF BIOMECHANICS, Bd. 43, Nr. 6, 1. April 2010 (2010-04-01), Seiten 1061-1066, beschreibt die Herstellung eines piezoelektrischen Aktorsubstrats, mit dem Osteoplasten mechanisch stimuliert werden, um die Bildung von neuem Knochengewebe zu beschleunigen.

Alexandre Poulin et al: "Dielectric elastomer actuator for mechanical loading of 2D cell cultures", LAB ON A CHIP, Bd. 16, Nr. 19, 1. Januar 2016 (2016-01-01), Seiten 3788-3794, offenbart einen deformierbaren Bioreaktor, mit dem humane lymphatische Endothelzellen mechanisch stimuliert werden können, wobei der Bioreaktor eine DEA-Membran und dehnbare Elektroden aufweist.

Karl Svennerstein et al: "Mechanical stimulation of epithelial cells using polypyrrole microactuators", LAB ON A CHIP, Bd. 11, Nr. 19, 1. Januar 2011 (2011-01-01), Seite 3287, beschreibt Mikroaktoren für biologische Zellen.

US 2012/200200 A1 beschreibt Aktoren auf Basis von elektroaktiven Polymeren sowie ein Verfahren zu deren Herstellung.

Siegfried Bauer et al: "Piezoelectric and Electrostrictive Polymers as EAPs: devices and Applications", 1. Januar 2016 (2016-01-01), ELECTROMECHANICALLY ACTIVE POLYMERS. POLYMERS AND POLYMERIC COMPOSITES: A REFERENCE SERIES. SPRINGER, CHAM, SPRINGER INTERNATIONAL PUBLISHING, DE, pages 533-547, beschreibt die Herstellung von EAPs mit Siebdruckverfahren.

DE 10 2011 107 046 A1 beschreibt mit Siebdruckverfahren hergestellte Piezomikropumpen aus P(VDF-TrFE). Diese werden in Lab-on-chip-Anwendungen eingesetzt.

Die DE 10 2011 075 127 A1 betrifft auf EAP hergestellte Mikropumpen für Lab-on-chip-Anwendungen.

Der vorliegenden Erfindung liegt daher ausgehend von dem Stand der Technik die Aufgabe zugrunde, ein Aktorsubstrat bereitzustellen, das sich mit geringen Kosten und in einem hohen Durchsatz fertigen lässt. Das Aktorsubstrat soll außerdem so gestaltet sein, dass es beliebig schwingen, vibrieren oder sich elastisch verformen kann und so zur gezielten Stimulierung von Zellkulturen verwendet werden kann.

Gelöst wird diese Aufgabe durch das Aktorsubstrat mit den Merkmalen des Patentanspruches 1 und dessen Verwendung gemäß Patentanspruch 13. Außerdem wird die Aufgabe durch das Verfahren zu dessen Herstellung mit den Schritten des Patentanspruches 9 gelöst.

Das erfindungsgemäße Aktorsubstrat für die Züchtung biologischer Zellen enthält mindestens eine Aktoreinheit mit einer oberen Elektrode und einer unteren Elektrode sowie einem zwischen den beiden Elektroden angeordneten und unmittelbar an die obere und untere Elektrode angrenzenden Aktorelement, das aus mindestens einer Schicht aus mindestens einem elektroaktiven Polymer (EAP) besteht, wobei die Elektroden als Schicht oder als Streifen angeordnet sind, jeweils an mindestens einer Stelle in einer parallel zur EAP-Schicht verlaufenden Richtung über die EAP- Schicht hinausragen und diese Überstände Kontaktstellen für eine Spannungsquelle darstellen.

Unter einem EAP werden im Rahmen dieser Erfindung alle Polymere verstanden, die durch Anlegen einer elektrischen Spannung ihre Form ändern. Zu dieser Gruppe werden elektrostriktive und piezoelektrische Polymere gezählt. Die Deformation von piezoelektrischen Polymeren ist proportional zur elektrischen Feldstärke, während die Deformation von elektrostriktiven Polymeren proportional zum Quadrat der elektrischen Feldstärke ist. Unter den Begriff des EAP fallen keine Kompositmaterialien aus magnetisierbaren oder elektrisch polarisierbaren Partikeln und einem Polymer als Matrixmaterial, wie MAP-Komposite, MRE-Komposite oder ERE-Komposite.

Wird eine Spannungsquelle an die Kontaktstellen angeschlossen, kann eine elektrische Spannung an der mindestens einen unteren und der mindestens einen oberen Elektrode erzeugt werden. Die mindestens eine, dazwischenliegende EAP-Schicht kann einem elektrischen Feld mit variabler Feldstärke aus- gesetzt werden. Auf jede Änderung in der Feldstärke des äußeren elektrischen Feldes reagiert die EAP-Schicht mit einer Expansion oder Kontraktion. Durch den Kontakt der EAP-Schicht zu der oberen und der unteren Elektrode wird eine Wölbung der gesamten Aktoreinheit hervorgerufen. Zusätzlich zu der ersten Aktoreinheit vorhandene Aktoreinheiten haben dabei einen verstärkenden Effekt. Je mehr Aktoreinheiten vorhanden sind, desto größer ist die Wölbung, die bei Anlegen einer bestimmten Spannung beobachtet wird.

Für den Fall, dass die Spannungsquelle mit Wechselspannung betrieben wird und die Elektroden als Schicht angeordnet sind, führt die Aktoreinheit eine Schwingung mit einer an die Wechselspannung-Frequenz angepassten Periode aus.

Bei der Ausgestaltung der Elektroden als Streifen, können obere und untere Elektrode so angeordnet werden, dass sie in der Projektion des Aktorsubstrates auf eine Ebene nur in einem kleinen Bereich überlappen. Wird an eine solche Elektrodenanordnung eine Gleichspannung angelegt, kann prinzipiell jede beliebige Oberflächentopographie des Aktorsubstrates erzeugt werden. Durch Anlegen einer Wechselspannung an eine solche Elektrodenanordnung kann das Aktorsubstrat eine Vibration in Form einer stehenden Wellen mit beliebig vielen Knotenpunkten ausführen.

Dadurch, dass die Elektroden über die EAP-Schicht hinausragen und Überstände bilden, kann bei dem erfindungsgemäßen Aktorsubstrat auf gesonderte Kontaktpunkte für die Spannungsquelle verzichtet werden. Die Überstände können selbst als Kontaktstellen für die Spannungsquelle dienen.

Das Aktorelement ist vorteilhafterweise aus einer Paste enthaltend ein gemäß ISO 10993 biokompatibles und/oder gegenüber Licht aus dem Wellenlängenbereich von 400 bis 1000 nm transparentes EAP, bevorzugt aus einer Paste enthaltend Polyvinylidendifluorid, besonders bevorzugt einer Paste enthaltend ein Poly-(vinylidendifluorid)-Copolymer oder ein Poly- (vinylidendifluorid)-Terpolymers, ganz besonders bevorzugt aus einer Paste enthaltend Poly-(Vinylidendifluorid-Trifluoroethylen) oder Poly-(Vinylidendifluorid-Trifluoroethylen-Trifluorovinylchlorid), siebgedruckt.

Die Biokompatibilität des EAP gemäß ISO 10993 kann dabei sicherstellen, dass das Aktorelement grundsätzlich die Voraussetzung für den Einsatz in der Medizintechnik erfüllt. Eine Verträglichkeit des Polymers mit biologischen Zellen, wie beispielsweise menschlichen und tierischen Zellen kann gewährleistet werden. Eine solche Verträglichkeit wird angenommen, wenn es keinen Hinweis auf eine abstoßende Immunantwort oder auf eine allergische Reaktion bei Kontakt des Polymers mit den biologischen Zellen gibt.

Die Verwendung eines gegenüber Licht aus dem Wellenlängenbereich von 400 bis 1000 nm transparenten EAP erhöht die Transparenz des Aktorsubstrates und erlaubt in Verbindung mit transparenten Elektroden die Charakterisierung des auf dem Aktorsubstrat wachsenden Zellgewebes mittels Lichtmikroskopie. So kann das Zellgewebe sogar während des Kultivierungsprozesses überwacht werden. Dies erlaubt es, krankhafte Zustände des Zellgewebes in einem frühen Stadium zu erkennen.

Von Transparenz des gesamten Aktorelementes wird im Sinne dieser Erfindung gesprochen, wenn es Licht aus dem Wellenlängenbereich von 400 bis 1000 nm zu einem Anteil von mindestens 50% durchlässt. Die Transparenz der einzelnen Bestandteile des Aktorelements sollte demnach mindestens genauso hoch sein. Das bedeutet, dass die Elektroden und die EAP-Schicht in dem Wellenlängenbereich von 400 nm bis 1000 nm einen Transmissionsgrad von 50% oder besser aufweisen sollten.

Da das Aktorelement nach dem Siebdruck getrocknet wird und die leichtflüchtigen Bestandteile der Paste verdampfen, kann das Aktorelement in dem Aktorsubstrat bevorzugt mindestens 99,0 % eines elektroaktiven Polymers ausgewählt aus der Gruppe bestehend aus Polyvinylidendifluorid, einem Poly- (vinylidendifluorid)-Copolymer einem Poly-(vinylidendifluorid)-Terpolymer, insbesondere Poly-(Vinylidendifluorid-Trifluoroethylen) oder Poly-(Vinylidendifluorid-Trifluoroethylen-Trifluorovinylchlorid) enthalten.

Polyvinylidendifluorid und die Copolymere des Polyvinylidendifluorids, wie beispielsweise Poly-(Vinylidendifluorid-Trifluoroethylen) können bei Anlegen eines elektrischen Feldes kontrahieren. Die Poly-(vinylidendifluorid)-Terpolymer dehnen sich mit zunehmender elektrischer Feldstärke hingegen bevorzugt aus.

In einer weiteren bevorzugten Ausführungsform des Aktorsubstrates sind die Elektroden aus einer Paste enthaltend ein elektrisch leitendes und gemäß ISO 10993 biokompatibles und/oder gegenüber Licht aus dem Wellenlängenbereich von 400 bis 1000 nm transparentes Polymer, bevorzugt aus einer Paste enthaltend [Poly-(3,4-Ethylendioxythiophen)]:PSS[poly(4-styrolsulfonat)] (PEDOT), siebgedruckt.

Ein Bestandteil der PEDOT:PSS-Paste kann dabei eine druckfähige Silber- oder Ruß-basierte Paste sein. Derartige Pasten sind beispielsweise kommerziell bei DuPont unter dem Namen DuPont 5000 oder DuPont 7102 erhältlich. Diese kommerziell erhältlichen Pasten weisen allerdings den Nachteil auf, dass sie für sichtbares Licht nicht transparent sind. Daher ist es bevorzugt, eine elektrisch leitfähige Paste auf Basis von Silber-Nanofäden für die Bereitstellung der PEDOT:PSS Paste zu benutzen.

Da auch die Elektroden nach dem Siebdrucken getrocknet werden und die leichtflüchtigen Anteile der Paste verdampfen, ist es bevorzugt, wenn die Elektroden mehr als 99,0% PEDOT:PSS enthalten.

Werden die Elektroden aus einem gemäß ISO 10993 biokompatiblen elektrisch leitfähigen Polymer hergestellt so kann bei geeigneter Auswahl des in der EAP-Schicht enthaltenen elektroaktiven Polymers das gesamte Aktorsubstrat ohne Bedenken in der Medizintechnik eingesetzt werden.

Die mindestens eine Aktoreinheit kann in einer weiteren Variante des Aktorsubstrates fixiert sein, insbesondere an einem Punkt, an mindestens zwei gegenüberliegenden Punkten oder an den Rändern der Aktoreinheit.

Durch die Fixierung der Aktoreinheit kann die Wölbung bzw. Auslenkung des Aktorsubstrates, die bei dem Anlegen einer Spannung erzielt wird, modifiziert werden. Die unter Einwirkung einer Wechselspannung angeregte Vibration kann durch die Art der Fixierung moduliert werden.

Bevorzugt ist, wenn das Aktorsubstrat in einem Frequenzbereich von 0,01 bis 40 KHz, insbesondere von 1 bis 20 KHz, vibrieren kann.

Dies kann dadurch erreicht werden, dass eine Wechselspannung mit einer entsprechend hohen Frequenz an die Kontaktstellen angelegt wird. Für die Vibration ist es von Vorteil, wenn das EAP in dem Aktorelement eine kurze Ansprechzeit hat, also schnell auf die Änderung der elektrischen Feldstärke reagiert. Des Weiteren ist es vorteilhaft, wenn das elektrisch leitfähige Polymer, aus dem die mindestens eine untere und mindestens eine obere Elektrode siebgedruckt sind, flexibel und elastisch ist, so dass die Elektroden der Bewegung bzw. Auslenkung der EAP-Schicht folgen können und keinen Wider- stand bieten oder zu einer Dämpfung führen.

Vorzugsweise weisen die oberen und unteren Elektroden eine Dicke von 10 nm bis 10 µm, besonders bevorzugt von 100 nm bis 400 nm, auf. Die EAP-Schichten weisen vorzugsweise eine Dicke von 10 nm bis 10 µm, besonders bevorzugt von 100 nm bis 1 µm, auf.

Fernerhin ist es bevorzugt, wenn auf der oberen und/oder unteren Elektrode mindestens eine elektrisch isolierende Schicht angeordnet ist.

Die elektrisch isolierende Schicht kann verhindern, dass die auf dem Aktorsubstrat angesiedelten Zellen einer hohen elektrischen Spannung ausgesetzt sind und dabei eventuell zerstört werden.

Das Aktorsubstrat kann auf einer Trägerfläche, bevorzugt auf einer PET-Folie, angeordnet sein.

Alternativ hierzu kann das Aktorsubstrat auch freitragend ohne Trägerfläche aufgehängt sein, da die PVDF- Folien eine ausreichende Stabilität hierfür bieten.

Das erfindungsgemäße Verfahren zur Herstellung des zuvor beschriebenen

Aktorsubstrates enthält folgende Verfahrensschritte:
(a) Formen und Abscheiden einer druckbaren Paste aus einem elektrisch leitfähigen Polymer mittels Siebdruck und Trocknen zu einer unteren Elektrode,
(b) Herstellen einer druckbaren Paste aus einem elektroaktiven Polymer durch Lösen des elektroaktiven Polymers in einer Mischung aus mindestens zwei Lösungsmitteln und anschließendes Entfernen eines Lösungs- mittels zu mindestens 90 Gew.-%,
(c) Formen und Abscheiden der druckbaren Paste aus dem elektroaktiven Polymer auf der unteren Elektrode mittels Siebdruck und Trocknen zu einer EAP-Schicht und
(d) Formen und Abscheiden einer druckbaren Paste aus einem elektrisch leitfähigen Polymer auf der von der unteren Elektrode abgewandten Seite der EAP-Schicht und Trocknen zu einer oberen Elektrode.

Das Siebdruckverfahren ist eine häufig angewandte und industriell eingesetzte Methode. Durch diese Methode können die Aktorsubstrate kostengünstig und in hohem Durchsatz hergestellt werden. Die Fertigung kann dabei sogar in einer Fertigungsstraße geschehen, die mehrere Druckstationen und Trocknungsbereiche aufweist und voll automatisiert ist.

Das Abscheiden der druckbaren Paste aus einem elektrisch leitfähigen Polymer erfolgt bevorzugt mit Hilfe eines Siebes, so dass die Elektrode entweder als Schicht oder als Streifen geformt werden kann.

Bevorzugt ist es, wenn das mindestens erste Lösungsmittel einen Siedepunkt von unter 150°C und/oder einen Dampfdruck von über 5 hPa (bei 20°C) aufweist und das mindestens eine zweite Lösungsmittel hochsiedend ist und einen um mindestens 50°C höheren Siedepunkt aufweist als das erste Lösungs- mittel und/oder dessen Siedepunkt so gewählt ist, dass die Lösungsmittel-Mischung einen Trennfaktor α von >1, aufweist.

Die mindestens zwei Lösungsmittel sind außerdem bevorzugt so gewählt, dass sie gesundheitlich unbedenklich und ungefährlich sind.

Des Weiteren ist es von Vorteil, wenn das Trocknen in den Verfahrensschritten (b) bis (d) bei einer Temperatur von 100 bis 140°C, bevorzugt bei einer Temperatur von 115 bis 125°C, erfolgt, wobei das Trocknen zu der unteren und der oberen Elektrode mindestens 4 Minuten, bevorzugt mindestens 5 Minuten, dauert und das Trocknen zu der EAP-Schicht mindestens 15 Minuten, bevorzugt mindestens 20 Minuten, dauert.

Eine weitere erfindungsgemäße Verfahrensalternative sieht vor, dass auf die obere Elektrode aus Verfahrensschritt (d) durch Siebdruck und Trocknen mindestens eine weitere EAP-Schicht und mindestens eine weitere Elektrode aufgebracht werden, wobei die mindestens eine weitere EAP-Schicht zuerst auf der oberen Elektrode abgeschieden wird.

Erfindungsgemäß kann das hier beschriebene Aktorsubstrat für eine in-vitro Züchtung biologischer Zellen verwendet werden. Die Zellkultivierung kann dadurch beschleunigt werden.

Bevorzugt können Zellen auf das Aktorsubstrat aufgebracht und über einen Zeitraum von 1 Stunde bis 4 Wochen gezüchtet werden, besonders bevorzugt unter Anlegen einer kontinuierlichen Wechselspannung oder modulierten Spannung an die Kontaktstellen.

Durch das Anlegen einer kontinuierlichen Wechselspannung oder modulierten Spannung kann beispielsweise die Migration von Zellen kontrolliert werden. Das Aktorsubstrat ändert fortlaufend seine Oberflächentopographie und exponiert einmal zellanziehende und einmal zellabstoßende Bereichen, die zu einer Bewegung der biologischen Zellen führen können, ohne diese in ihrem natürlichen Zellzyklus zu stören.

Durch das Anlegen einer kontinuierlichen Wechselspannung oder modulierten Spannung kann es aber auch zu einer gezielten Differenzierung von Stammzellen führen. So könnten in-vitro verschiedene spezielle Zelltypen aus Stammzellen gezüchtet werden.

Außerdem ist es bevorzugt, wenn die EAP-Schicht vor dem Aufbringen der biologischen Zellen mit Hilfe eines Wechselspannungspulses mit einer

Amplitude von mindestens 150 V pro µm EAP- Schichtdicke und einer Frequenz von mindestens 10 Hz für mindestens 10 Sekunden gepolt wird.

Die Polung ist insbesondere bei den Aktorsubstraten notwendig, bei denen ein piezoelektrisches Polymer als EAP verwendet wird. Das Polyvinylidendifluorid-Copolymer Poly-(Vinylidendifluorid-Trifluoroethylen) muss beispielsweise in einem Polschritt gepolt werden, um es einsatzbereit zu machen.

Das erfindungsgemäße Aktorsubstrat kann ebenso in in-vivo Verfahren genutzt werden. Bevorzugt wird es dabei mit Zellen besiedelt und als aktives Körperimplantat verwendet. Besonders bevorzugt wird es in Verfahren zur Beschleunigung von Wundheilung und Wundschließung sowie Knochenheilung benutzt.

Die Erfindung soll nachfolgend anhand der Figuren näher erläutert werden, ohne diese auf die hier dargestellten spezifischen Ausführungsformen einschränken zu wollen.

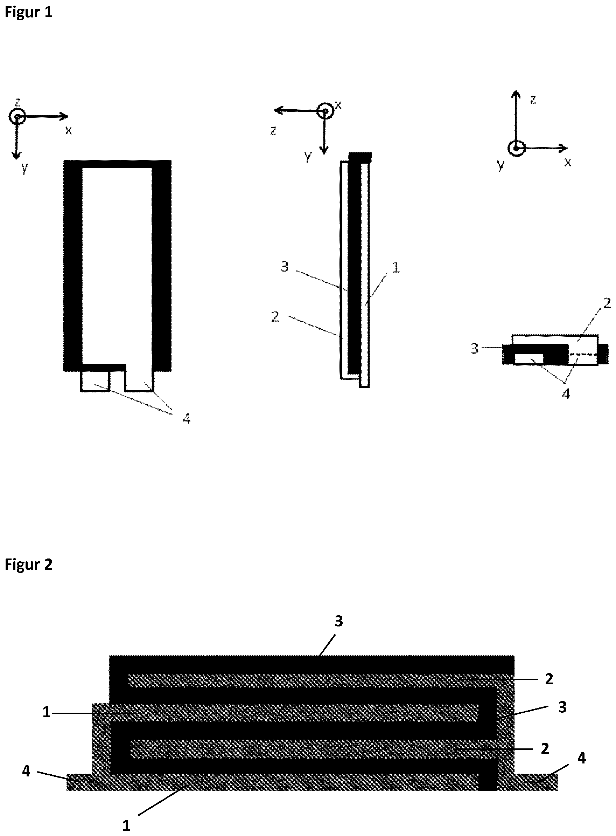

Figur 1 zeigt eine schematische Darstellung des erfindungsgemäßen Aktorsubstrates in einer Projektion in allen drei Ebenen (links: Aufsicht, Mitte: Seitenansicht, rechts: Frontansicht). Die Elektroden sind hier als Schicht geformt. Die untere Elektrode (1) und die obere Elektrode (2) ragen jeweils versetzt an einer einzigen Stelle über die EAP-Schicht (3) hinaus und bilden einen Überstand (4). Dieser Überstand (4) dient als Kontaktstelle für die Spannungsquelle. Die obere Elektrode ist zudem abgeknickt, so dass der Überstand (4) der oberen Elektrode in der x/z-Projektion des Aktorsubstrates auf der gleichen Höhe wie der Überstand (4) der unteren Elektrode liegt.

Der Überstand ist in Figur 2 noch einmal detaillierter gezeigt. Durch den Überstand (4), der als Kontaktestelle für die Spannungsquelle dient, wird bei mehreren Aktoreinheiten hier ein fingerförmiger Verlauf der oberen Elektroden (2) sowie einen fingerförmiger Verlauf der unteren Elektroden (1) generiert. Die EAP-Schicht (3) weist hingegen bei dem Aktorsubstrat aus mehreren Aktoreinheiten einen mäanderförmigen Verlauf auf.

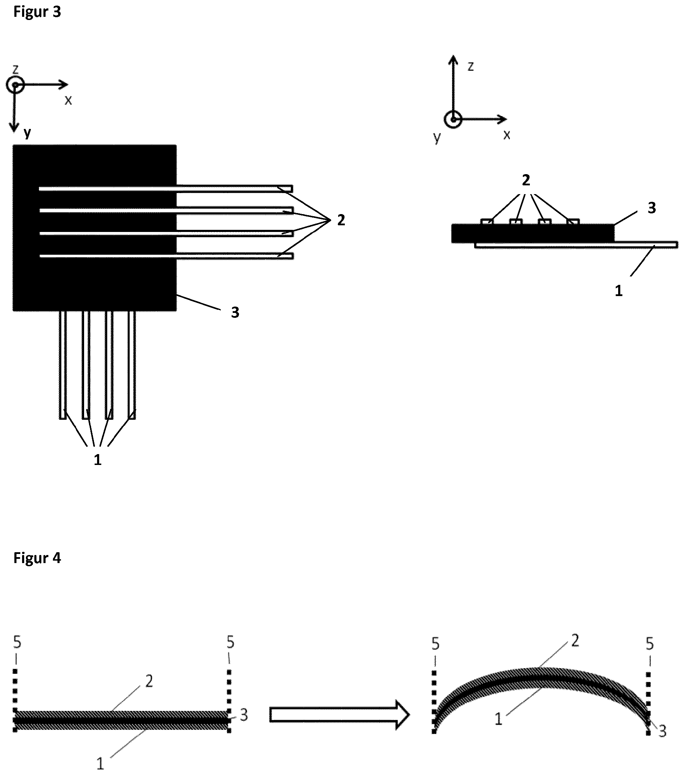

Figur 3 ist eine schematische Ansicht eines weiteren erfindungsgemäßen Aktorsubstrates in der Projektion in zwei verschiedene Ebenen (links: Aufsicht, rechts: Seitenansicht). Die unteren und oberen Elektroden (1,2) sind hier als Streifen geformt. In der x/y-Projektion der Aufsicht ergeben sich somit nur punktuelle Überlappbereiche der verdeckten unteren Elektroden (1) mit den auf der EAP-Schicht aufliegenden oberen Elektroden (2).

In Figur 4 zeigt die Reaktion einer Aktoreinheit auf das Anlegen einer elektrischen Spannung und die Änderung der elektrischen Feldstärke gezeigt (links: vor Änderungen der Feldstärke, rechts: nach Änderung der Feldstärke). Die Aktoreinheit bestehend aus unterer Elektrode (1), EAP-Schicht (3) und oberer Elektrode (2) ist hier an beiden Rändern mit Hilfe von Fixpunkten (5) eingespannt. Eine Expansion der EAP-Schicht (2) führt somit zwangsläufig zu einer Wölbung des gesamten Aktorsubstrates (rechte schematische Abbildung).

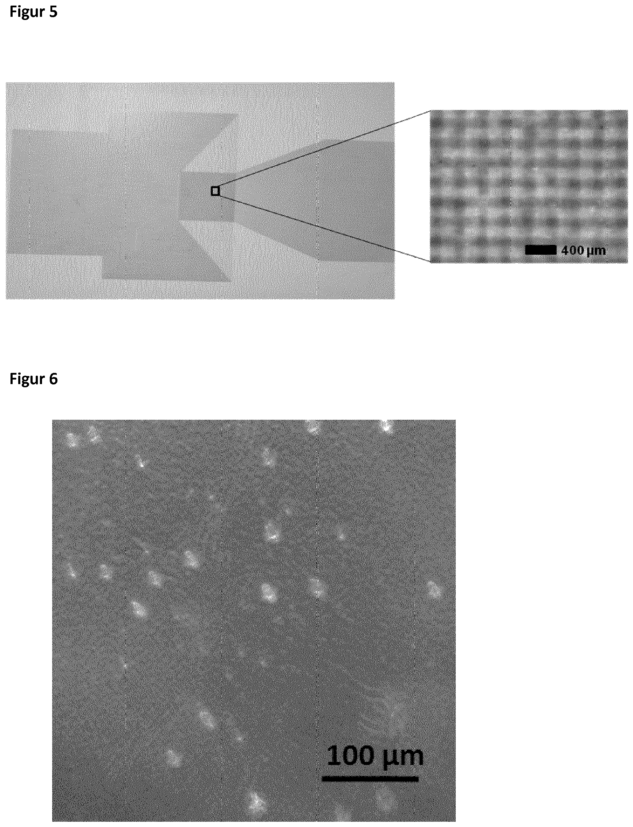

Figur 5 zeigt zwei Aufnahmen eines erfindungsgemäßen Aktorsubstrates (links: großer Ausschnitt, rechts: vergrößerter Ausschnitt). Das Aktorsubstrat entspricht dem in Figur 3 gezeigten Schema. In der vergrößerten Aufnahme ist zu erkennen, dass die oberen und unteren Elektroden streifenförmig als Pedot:PSS-Elektrodenlinien ausgebildet sind. Die unteren Elektrodenlinien sind dabei um 90° zu den oberen Elektrodenlinien gedreht und bilden in einer Aufsicht auf das Aktorsubstrat Kreuzungspunkte aus. Die Kreuzungspunkte sind dabei auf einem gedachten regelmäßigen Gitter angeordnet.

In Figur 6 ist ein Zweikanal-Durchlicht-Fluoreszensbild von GFPexprimierenden Dictyostelium Discoideum Zellen mikroskopiert, das auf einem Aktorsubstrat aufgewachsen ist. Hiermit wird demonstriert, dass das Aktorsubstrat die Untersuchung von wachsendem Zellgewebe mit Hilfe klassischer Lichtmikroskopiemethoden erlaubt.

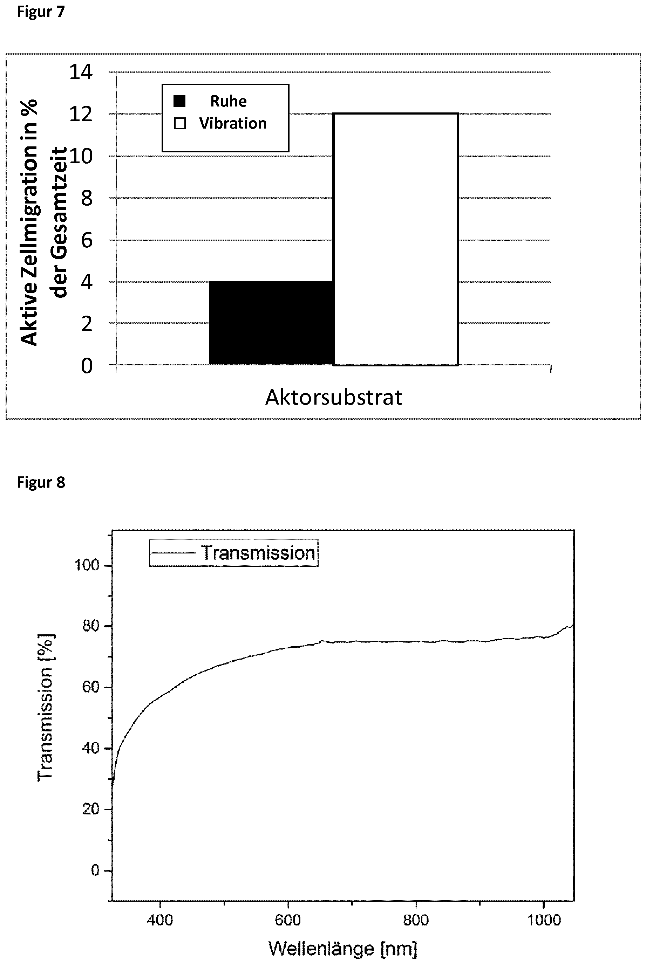

Figur 7 zeigt ein Diagramm, in dem der Anteil der aktiven Migrationsphasen von Dictyostelium Discoideum Zellen auf einem Aktorsubstrat einmal mit und einmal ohne Vibrationsstimulation erfasst ist. Die Vibration wurde mit einer Wechselspannung von 1500 Volt Spitze-Spitze bei 100 Hz erzeugt. Die aktiven Migrationsphasen wurden über einen Zeitraum von 2 Stunden gemessen. Die Zellen, die unter Vibrationsstimulation gezüchtet werden zeigen dabei eine nachweisbar höhere Migrationsaktivität.

In dem Diagramm der Figur 8 ist das Transmissionsspektrum des Aktorsubstrates in Abhängigkeit der Wellenlänge gezeigt. Es ist zu sehen, dass der Transmissionsgrad im Wellenlängenbereich von 400 bis 1000 nm mindestens 50% beträgt.

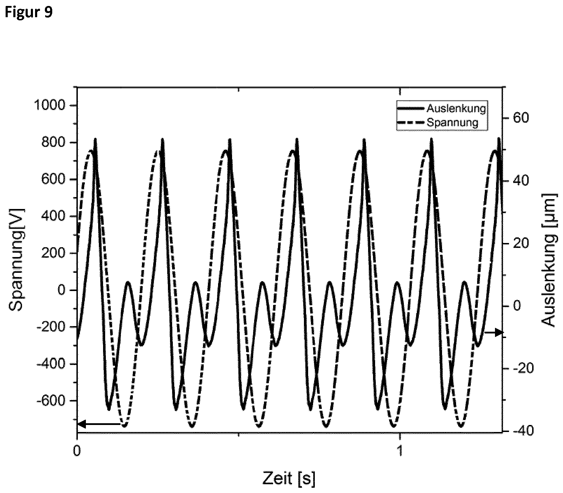

Figur 9 zeigt ein Diagramm, in dem sowohl die Auslenkung des Aktorsubstrates als auch die am Aktorsubstrat anliegende Spannung gegen die Zeit aufgetragen sind. Dabei wird deutlich, dass die EAP-Schicht des Aktorsubstrates aus einer elektrostriktiven Polymerschicht mit einer sehr kurzen Ansprechzeit besteht. Das Aktorsubstrat reagiert schnell auf die Änderungen der elektrischen Feldstärke, was daran zu erkennen ist, dass die Maxima der Auslenkung nur um etwa 20 Millisekunden zu den Maxima bzw. Minima phasenverschoben sind.

## Patentansprüche

1. Aktorsubstrat für die Züchtung biologischer Zellen
enthaltend mindestens eine Aktoreinheit mit einer oberen Elektrode und einer unteren Elektrode sowie einem zwischen den beiden Elektroden angeordneten und unmittelbar an die obere und untere Elektrode angrenzenden Aktorelement, das aus mindestens einer Schicht aus mindestens einem elektroaktiven Polymer (EAP) besteht, wobei die Elektroden als Schicht oder als Streifen angeordnet sind, jeweils an mindestens einer Stelle in einer parallel zur EAP-Schicht verlaufenden Richtung über die EAP-Schicht hinausragen und diese Überstände Kontaktstellen für eine Spannungsquelle darstellen.

2. Aktorsubstrat nach Anspruch 1,
**dadurch gekennzeichnet, dass** das Aktorelement aus einer Paste enthaltend ein gemäß ISO 10993 biokompatibles und gegenüber Licht aus dem Wellenlängenbereich von 400 bis 1000 nm transparentes EAP, bevorzugt aus einer Paste enthaltend Polyvinylidendifluorid, besonders bevorzugt einer Paste enthaltend ein Poly-(vinylidendifluorid)-Copolymer oder ein Poly-(vinylidendifluorid)-Terpolymers, ganz besonders bevorzugt aus einer Paste enthaltend Poly-(Vinylidendifluorid-Trifluoroethylen) oder Poly-(Vinylidendifluorid-Trifluoroethylen-Trifluorovinylchlorid), siebgedruckt ist.

3. Aktorsubstrat nach einem der vorhergehenden Ansprüche,
**dadurch gekennzeichnet, dass** die Elektroden aus einer Paste enthaltend ein elektrisch leitendes und gemäß ISO 10993 biokompatibles und gegenüber Licht aus dem Wellenlängenbereich von 400 bis 1000 nm transparentes Polymer, bevorzugt aus einer Paste enthaltend [Poly-(3,4-Ethylendioxythiophen)]:PSS[poly(4-styrolsulfonat)] (PEDOT), siebgedruckt sind.

4. Aktorsubstrat nach einem der vorhergehenden Ansprüche,
**dadurch gekennzeichnet, dass** die mindestens eine Aktoreinheit fixiert ist, insbesondere an einem Punkt, an mindestens zwei gegenüberliegenden Punkten oder an den Rändern der Aktoreinheit.

5. Aktorsubstrat nach einem der vorhergehenden Ansprüche,
**dadurch gekennzeichnet, dass** das Aktorsubstrat in einem Frequenzbereich von 0,01 bis 40 KHz, insbesondere von 1 bis 20 KHz, vibrationsfähig ist.

6. Aktorsubstrat nach einem der vorhergehenden Ansprüche,
**dadurch gekennzeichnet, dass** die oberen und unteren Elektroden eine Dicke von 10 nm bis 10 µm, bevorzugt von 100 nm bis 400 nm, aufweisen und die EAP-Schichten ein Dicke von 10 nm bis 10 µm, bevorzugt von 100 nm bis 1 µm aufweisen.

7. Aktorsubstrat nach einem der vorhergehenden Ansprüche,
**dadurch gekennzeichnet, dass** auf der oberen und/oder unteren Elektrode mindestens eine elektrisch isolierende Schicht angeordnet ist.

8. Aktorsubstrat nach einem der vorhergehenden Ansprüche,
**dadurch gekennzeichnet, dass** das Aktorsubstrat entweder auf einer Trägerfläche, bevorzugt auf einer PET-Folie, angeordnet ist.

9. Verfahren zur Herstellung des Aktorsubstrates nach den Ansprüchen 1 bis 8, enthaltend folgende Verfahrensschritte:
(a) Formen und Abscheiden einer druckbaren Paste aus einem elektrisch leitfähigen Polymer mittels Siebdruck und Trocknen zu einer unteren Elektrode,
(b) Herstellen einer druckbaren Paste aus einem elektroaktiven Polymer durch Lösen des elektroaktiven Polymers in einer Mischung aus mindestens zwei Lösungsmitteln und anschließendes Entfernen eines Lösungsmittels zu mindestens 90 Gew.-%,
(c) Formen und Abscheiden der druckbaren Paste aus dem elektroaktiven Polymer auf der unteren Elektrode mittels Siebdruck und Trocknen zu einer EAP-Schicht und
(d) Formen und Abscheiden einer druckbaren Paste aus einem elektrisch leitfähigen Polymer auf der von der unteren Elektrode abgewandten Seite der EAP-Schicht und Trocknen zu einer oberen Elektrode.

10. Verfahren nach Anspruch 9, **dadurch gekennzeichnet, dass** das mindestens eine erste Lösungsmittel einen Siedepunkt von unter 150°C und/oder einen Dampfdruck von über 5 hPa (bei 20°C) aufweist und das mindestens eine zweite Lösungsmittel hochsiedend ist und einen um mindestens 50°C höheren Siedepunkt aufweist als das erste Lösungsmittel und/oder dessen Siedepunkt so gewählt ist, dass die Lösungsmittel-Mischung einen Trennfaktor α von >1, aufweist.

11. Verfahren nach einem der Ansprüche 9 oder 10, **dadurch gekennzeichnet, dass** das Trocknen in den Verfahrensschritten (b) bis (d) bei einer Temperatur von 100 bis 140°C, bevorzugt bei einer Temperatur von 115 bis 125°C, erfolgt, wobei das Trocknen zu der unteren und der oberen Elektrode mindestens 4 Minuten, bevorzugt mindestens 5 Minuten, dauert und das Trocknen zu der EAP-Schicht mindestens 15 Minuten, bevorzugt mindestens 20 Minuten, dauert.

12. Verfahren nach einem der Ansprüche 9 bis 11, **dadurch gekennzeichnet, dass** auf die obere Elektrode aus Verfahrensschritt (d) durch Siebdruck und Trocknen mindestens eine weitere EAP-Schicht und mindestens eine weitere Elektrode aufgebracht werden, wobei die mindestens eine weitere EAP-Schicht auf der oberen Elektrode zuerst abgeschieden wird.

13. Verwendung des Aktorsubstrates nach den Ansprüchen 1 bis 8 als Substrat für eine in-vitro Züchtung biologischer Zellen.

14. Verwendung nach dem vorhergehenden Anspruch, bei der die biologischer Zellen auf das Aktorsubstrat aufgebracht und über einen Zeitraum von 1 Stunde bis 4 Wochen gezüchtet werden, bevorzugt unter Anlegen einer kontinuierlichen Wechselspannung oder modulierten Spannung an die Kontaktstellen.

15. Verwendung nach dem vorhergehenden Anspruch, bei der die EAP-Schicht vor dem Aufbringen der biologischen Zellen mit Hilfe eines Wechselspannungspulses mit einer Amplitude von mindestens 150 V pro µm EAP- Schichtdicke und einer Frequenz von mindestens 10 Hz für mindestens 10 Sekunden gepolt wird.

## Claims

1. An actuator substrate for the cultivation of biological cells,
comprising at least one actuator unit, including an upper electrode and a lower electrode and an actuator element, which is arranged between the two electrodes and directly adjoins the upper and lower electrode and which is composed of at least one layer made of at least one electroactive polymer (EAP), the electrodes being arranged in the form of a layer or strips, protruding respectively beyond the EAP layer in at least one location in a direction extending parallel to the EAP layer, and these protrusions representing contact points for a voltage source.

2. The actuator substrate according to claim 1,
**characterized in that** the actuator element is screen-printed from a paste comprising an EAP that is biocompatible according to ISO 10993 and transparent with respect to light from the wavelength range of 400 to 1000 nm, preferably from a paste comprising polyvinylidene difluoride, particularly preferably a paste comprising a poly(vinylidene difluoride) copolymer or a poly(vinylidene difluoride) terpolymer, most particularly preferably from a paste comprising poly(vinylidene difluoride-trifluoroethylene) or poly(vinylidene difluoride-trifluoroethylene-trifluorovinyl chloride).

3. The actuator substrate according to any one of the preceding claims,
**characterized in that** the electrodes are screen-printed from a paste comprising an electrically conducting polymer that is biocompatible according to ISO 10993 and transparent with respect to light from the wavelength range of 400 to 1000 nm, preferably from a paste comprising [poly(3,4-ethylenedioxythiophene)]:PSS[poly(4-styrenesulfonate)] (PEDOT).

4. The actuator substrate according to any one of the preceding claims,
**characterized in that** the at least one actuator unit is fixed, in particular at one point, at at least two opposing points, or at the edges of the actuator unit.

5. The actuator substrate according to any one of the preceding claims,
**characterized in that** the actuator substrate is vibration-capable in a frequency range of 0.01 to 40 KHz, in particular of 1 to 20 KHz.

6. The actuator substrate according to any one of the preceding claims,
**characterized in that** the upper and lower electrodes have a thickness of 10 nm to 10 µm, preferably of 100 nm to 400 nm, and the EAP layers have a thickness of 10 nm to 10 µm, preferably of 100 nm to 1 µm.

7. The actuator substrate according to any one of the preceding claims,
**characterized in that** at least one electrically insulating layer is arranged on the upper and/or lower electrode.

8. The actuator substrate according to any one of the preceding claims,
**characterized in that** the actuator substrate is arranged on a carrier surface, preferably on a PET film.

9. A method for producing the actuator substrate according to claims 1 to 8, comprising the following method steps:
(a) forming and depositing a printable paste made of an electrically conductive polymer by means of screen printing and drying to form a lower electrode;
(b) producing a printable paste from an electroactive polymer by dissolving the electroactive polymer in a mixture made of at least two solvents, and subsequently removing at least 90 wt.% of one solvent;
(c) forming and depositing the printable paste made of the electroactive polymer on the lower electrode by means of screen printing, and drying to form an EAP layer; and
(d) forming and depositing a printable paste made of an electrically conductive polymer onto the side of the EAP layer facing away from the lower electrode, and drying to form an upper electrode.

10. The method according to claim 9, **characterized in that** the at least one first solvent has a boiling point of less than 150°C and/or a vapor pressure of more than 5 hPa (at 20°C), and the at least one second solvent boils at high temperatures and has a boiling point that is at least 50°C higher than that of the first solvent and/or the boiling point of which is selected in such a way that the solvent mixture has a separation factor a of > 1.

11. The method according to either claim 9 or 10, **characterized in that** the drying in method steps (b) to (d) is carried out at a temperature of 100 to 140°C, preferably at a temperature of 115 to 125°C, the drying to form the lower and upper electrode lasting at least 4 minutes, preferably at least 5 minutes, and the drying to form the EAP layer lasting at least 15 minutes, preferably at least 20 minutes.

12. The method according to any one of claims 9 to 11, **characterized in that** at least one further EAP layer and at least one further electrode are applied to the upper electrode from method step (d) by means of screen printing and drying, the at least one further EAP layer being deposited first onto the upper electrode.

13. Use of the actuator substrate according to claims 1 to 8 as a substrate for an in-vitro cultivation of biological cells.

14. Use according to the preceding claim, wherein the biological cells are applied to the actuator substrate and cultivated over a period of 1 hour to 4 weeks, preferably while applying a continuous alternating voltage or modulated voltage at the contact points.

15. Use according to the preceding claim, wherein the EAP layer, prior to the application of the biological cells, is poled for at least 10 seconds by way of an AC voltage pulse having an amplitude of at least 150 V per µm EAP layer thickness and a frequency of at least 10 Hz.

## Revendications

1. Substrat d'actionneur pour la culture de cellules biologiques
contenant au moins une unité d'actionneur avec une électrode supérieure et une électrode inférieure ainsi qu'un élément d'actionneur disposé entre les deux électrodes et directement adjacent à l'électrode supérieure et inférieure, qui est constitué d'au moins une couche composée d'au moins un polymère électro-actif (EAP), dans lequel les électrodes sont disposées en tant que couche ou en tant que bande, font saillie de la couche d'EAP respectivement à au moins un emplacement dans une direction s'étendant parallèlement à la couche d'EAP, et ces parties saillantes représentent des emplacements de contact pour une source de tension.

2. Substrat d'actionneur selon la revendication 1,
**caractérisé en ce que** l'élément d'actionneur est sérigraphié à partir d'une pâte contenant un EAP biocompatible selon ISO 10993 et transparent à la lumière de la gamme de longueurs d'onde de 400 à 1000 nm, de préférence à partir d'une pâte contenant du difluorure de polyvinylidène, de manière particulièrement préférée une pâte contenant un copolymère de poly-(difluorure de vinylidène) ou un terpolymère de poly-(difluorure de vinylidène), mieux encore d'une pâte contenant du poly-(difluorure de vinylidène-trifluoroéthylène) ou poly(difluorure de vinylidène-trifluoroéthylène-trifluorochlorure de vinyle).

3. Substrat d'actionneur selon l'une quelconque des revendications précédentes,
**caractérisé en ce que** les électrodes sont sérigraphiées à partir d'une pâte contenant un polymère électroconducteur et biocompatible selon ISO 10993 et transparent à une lumière provenant de la gamme de longueurs d'onde de 400 à 1000 nm, de préférence à partir d'une pâte contenant [poly-(3,4-éthylènedioxythiophène)]:PSS[poly(4-styrène sulfonate)] (PEDOT).

4. Substrat d'actionneur selon l'une quelconque des revendications précédentes,
**caractérisé en ce que** la au moins une unité d'actionneur est fixée, en particulier sur un point, sur au moins deux points opposés ou sur les bords de l'unité d'actionneur.

5. Substrat d'actionneur selon l'une quelconque des revendications précédentes,
**caractérisé en ce que** le substrat d'actionneur est susceptible de vibrer dans une plage de fréquences de 0,01 à 40 KHz, en particulier de 1 à 20 KHz.

6. Substrat d'actionneur selon l'une quelconque des revendications précédentes,
**caractérisé en ce que** les électrodes supérieure et inférieure présentent une épaisseur de 10 nm à 10 µm, de préférence de 100 nm à 400 nm, et les couches d'EAP présentent une épaisseur de 10 nm à 10 µm, de préférence de 100 nm à 1 µm.

7. Substrat d'actionneur selon l'une quelconque des revendications précédentes,
**caractérisé en ce qu'**au moins une couche électriquement isolante est disposée sur l'électrode supérieure et/ou inférieure.

8. Substrat d'actionneur selon l'une quelconque des revendications précédentes,
**caractérisé en ce que** le substrat d'actionneur est disposé soit sur une surface de support, de préférence sur une feuille en PET.

9. Procédé pour produire le substrat d'actionneur selon les revendications 1 à 8, contenant les étapes de procédé suivantes :
(a) la formation et le dépôt d'une pâte imprimable composée d'un polymère électro-conducteur par sérigraphie et séchage pour obtenir une électrode inférieure,
(b) la production d'une pâte imprimable composée d'un polymère électro-actif par dissolution du polymère électro-actif dans un mélange composé d'au moins deux solvants et ensuite élimination d'un solvant à au moins 90 % en poids,
(c) la formation et le dépôt de la pâte imprimable composée du polymère électro-actif sur l'électrode inférieure par sérigraphie et séchage pour obtenir une couche d'EAP et
(d) la formation et le dépôt d'une pâte imprimable composée d'un polymère électro-conducteur sur la face de la couche d'EAP opposée à l'électrode inférieure et le séchage pour obtenir une électrode supérieure.

10. Procédé selon la revendication 9, **caractérisé en ce que** le au moins un premier solvant présente un point d'ébullition inférieur à 150 °C et/ou une pression de vapeur supérieure à 5 hPa (à 20 °C) et le au moins un deuxième solvant est à haut point d'ébullition et présente un point d'ébullition plus grand d'au moins 50 °C que le premier solvant et/ou le point d'ébullition de celui-ci est choisi de sorte que le mélange de solvants présente un facteur de séparation α > 1.

11. Procédé selon l'une quelconque des revendications 9 ou 10, **caractérisé en ce que** le séchage dans les étapes de procédé (b) à (d) s'effectue à une température de 100 à 140 °C, de préférence à une température de 115 à 125 °C, dans lequel le séchage pour obtenir l'électrode inférieure et l'électrode supérieure dure au moins 4 minutes, de préférence au moins 5 minutes, et le séchage pour obtenir la couche d'EAP dure au moins 15 minutes, de préférence au moins 20 minutes.

12. Procédé selon l'une quelconque des revendications 9 à 11, **caractérisé en ce qu'**au moins une autre couche d'EAP et au moins une autre électrode sont appliquées sur l'électrode supérieure de l'étape de procédé (d) par sérigraphie et séchage, dans lequel la au moins une autre couche d'EAP est tout d'abord déposée sur l'électrode supérieure.

13. Utilisation du substrat d'actionneur selon les revendications 1 à 8 comme substrat pour une culture in-vitro de cellules biologiques.

14. Utilisation selon la revendication précédente, pour laquelle les cellules biologiques sont appliquées sur le substrat d'actionneur et cultivées pendant une période de 1 heure à 4 semaines, de préférence sous application d'une tension alternative continue ou tension modulée aux emplacements de contact.

15. Utilisation selon la revendication précédente, pour laquelle la couche d'EAP, avant l'application des cellules biologiques, est polarisée à l'aide d'une impulsion de tension alternative avec une amplitude d'au moins 150 V par µm d'épaisseur de couche d'EAP et une fréquence d'au moins 10 Hz pendant au moins 10 secondes.
